# FASCICULE DE BREVET EUROPEEN

(11) **EP 1 601 363 B1**
(45) Date de publication et mention de la délivrance du brevet: **20.06.2012**
(21) Numéro de dépôt: 04717660.7
(22) Date de dépôt: 05.03.2004
(51) Int. Cl.: A61K 31/575, C07J 9/00, A61P 25/00

(54) **APPLICATION A TITRE DE MEDICAMENTS DE DERIVES DE CHOLEST-4-EN-3- ONE, COMPOSITIONS PHARMACEUTIQUES LES RENFERMANT ET NOUVEAUX DERIVES**
VERWENDUNG ALS ARZNEIMITTEL VON CHOLEST-4-EN-3-ON-DERIVATEN, SIE ENTHALTENDES MEDIKAMENT SOWIE NEUE DERIVATE
USE OF DERIVATIVES OF CHOLEST-4-EN-3-ONE AS MEDICAMENTS, PHARMACEUTICAL COMPOSITIONS CONTAINING SAME, NOVEL DERIVATIVES AND PREPARATION METHOD THEREOF

(30) Priorité: 11.03.2003 FR 0302992; 26.09.2003 FR 0311324
(43) Date de publication de la demande: 07.12.2005
(73) Titulaire: Trophos, 13288 Marseille Cedex 9 (FR)
(72) Inventeur: BORDET, Thierry, F-13009 Marseille (FR); DROUOT, Cyrille, F-13009 Marseille (FR); BUISSON, Bruno, F-13004 Marseille (FR)
(74) Mandataire: Galup, Cédric Olivier Nicolas
(86) Numéro de dépôt international: PCT/FR2004/000532
(87) Numéro de publication internationale: WO 2004/082581

(56) Documents cités:
- EP-A- 0 442 350
- HORITA, KIYOSHI ET AL: "Anti-arrhythmia constituent in Herba leonuri" NATURAL MEDICINES (TOKYO, JAPAN) , 56(5), 212-214 CODEN: NMEDEO; ISSN: 1340-3443, 2002, XP008033780
- YANG, Y. ET AL: "Anti-emetic principles of Pogostemon cablin" PHYTOMEDICINE , 6(2), 89-93 CODEN: PYTOEY; ISSN: 0944-7113, 1999, XP008033811
- KOLAK, UFUK ET AL: "Cardioactive diterpenoids from the roots of Salvia amplexicaulis" PLANTA MEDICA , 67(8), 761-763 CODEN: PLMEAA; ISSN: 0032-0943, 2001, XP008033781
- SUZUKI K ET AL: "The cholesterol metabolite cholest-4-en-3-one and its 3-oxo derivatives suppress body weight gain, body fat accumulation and serum lipid concentration in mice" BIOORGANIC & MEDICINAL CHEMISTRY LETTERS, OXFORD, GB, vol. 8, no. 16, 18 août 1998 (1998-08-18), pages 2133-2138, XP004137233 ISSN: 0960-894X
- KANEKO, EMI ET AL: "Induction of Intestinal ATP-binding Cassette Transporters by a Phytosterol-derived Liver X Receptor Agonist" JOURNAL OF BIOLOGICAL CHEMISTRY , 278(38), 36091-36098 CODEN: JBCHA3; ISSN: 0021-9258, 2003, XP001199518
- LIN, WEI-YU ET AL: "Anti-platelet aggregation and chemical constituents from the rhizome of Gynura japonica" PLANTA MEDICA , 69(8), 757-764 CODEN: PLMEAA; ISSN: 0032-0943, 2003, XP008033801
- DATABASE CAPLUS CHEMICAL ABSTRACT SERVICE, COLUMBUS; 29 janvier 2002 (2002-01-29), KAWAHARA, TOMIO ET AL: "Preparation of ergostenols and neuron projection regenerants" XP002292135 & JP 2002 030096 A2 (KYORIN PHARMACEUTICAL CO., LTD., JAPAN) 29 janvier 2002 (2002-01-29)
- UENSEREN E: "GAMMA IRRADIATION OF CHOLESTENONE OXIMES" INIS ATOMINDEX, INTERNATIONAL ATOMIC ENERGY AGENCY, VIENNA, AT, vol. 8, no. 6, 1977, pages 1-21,4PAGES, XP001164118 ISSN: 0004-7139
- KOBAYASHI M ET AL: "POLYPHOSPHORIC ACID-CATALYZED BECKMANN REARRANGEMENT OF 3-KETO-STEROID OXIMES" CHEMICAL AND PHARMACEUTICAL BULLETIN, TOKYO, JP, vol. 17, no. 6, 1969, pages 1255-1260, XP008029833 ISSN: 0009-2363 cité dans la demande
- AHMAD, M.S.; SIDDIQUI, A.H. ET AL: "Azasteroid from cholest-4,6-dien-3-one" AUSTRALIAN JOURNAL OF CHEMISTRY, vol. 22, no. 1, 1969, pages 271-274, XP008033857 cité dans la demande
- EVANS L.K.; GILLAM A.E.: "Effect of molecular environment on the absorption spectra of organic compounds in solution. III. Compounds containing the chromophore C:C C :N", JOURNAL OF THE CHEMICAL SOCIETY, 1943, pages 565-571, XP008092705,
- H.Allain, D. Bentue-Ferrer, O. Zékri: "Neuroprotection: définitions, limites et perspectives", , 2 January 1999 (1999-01-02), Retrieved from the Internet: URL:http://www.med.univ-rennes1.fr/etud/ph armaco/neuroprotection.htm [retrieved on 2008-10-17]
- Guégan Christelle, Sola Brigitte: "ETUDE DE L'APOPTOSE ET DU ROLE NEUROPROTECTEUR DU «NERVE GROWTH FACTOR» (NGF) LORS D'UNE ISCHEMIE CEREBRALE FOCALE PERMANENTE CHEZ LA SOURIS = APOPTOSIS AND NEUROPROTECTIVE EFFECT OF THE NERVE GROWTH FACTOR (NGF) IN A MODEL OF FOCAL PERMANENT CEREBRAL ISCHEMIA IN MICE", , 31 December 1999 (1999-12-31), Retrieved from the Internet: URL:http://cat.inist.fr/?aModele=afficheN& cpsidt=197578 [retrieved on 2008-10-17]
- "Riluzole", BIAM , 19 December 1996 (1996-12-19), Retrieved from the Internet: URL:http://www.biam2.org/www/Sub5121.html [retrieved on 2008-10-17]

## Description

La présente invention concerne l'application à titre de médicaments de dérivés de cholest-4-èn-3-one, notamment comme neuroprotecteurs par exemple dans les pathologies et les traumatismes liés à la dégénérescence ou à la mort des motoneurones, les compositions pharmaceutiques les renfermant, de nouveaux dérivés et leur procédé de préparation.

Les processus neurodégénératifs sont caractérisés par le dysfonctionnement et la mort des neurones entraînant la perte des fonctions neurologiques médiées par le cerveau (système nerveux central, SNC), la moelle épinière et le système nerveux périphérique (SNP). Ils peuvent résulter, entre autres, de situations pathologiques regroupées sous le terme de maladies ou affections neurodégénératives, de traumatisme, ou d'exposition à des toxines.

Les pathologies les plus importantes qui sont caractérisées par un processus dégénératif sont :
- les maladies chroniques neurodégénératives, héréditaires ou sporadiques, notamment la maladie d'Alzheimer, la maladie de Huntington, la maladie de Parkinson, la sclérose latérale amyotrophique, les amyotrophies spinales, la maladie de Creutzfeldt-Jakob, la sclérose en plaque, l'adrénoleucodystrophie, l'épilepsie, les démences, la schizophrénie, et les syndromes neurologiques associés au SIDA;
- les lésions neuronales liées au vieillissement ;
- les neuropathies périphériques héréditaires ou lésionnelles, comme les maladies de Fabry, de Charcot-Marie-Tooth, de Krabbe, les leucodystrophies, les neuropathies diabétiques et celles induites par les traitements anti-cancéreux ;
- les traumatismes du cerveau, des nerfs périphériques ou de la moelle épinière;
- les ischémies du cerveau ou de la moelle épinière suite à un accident cérébro-vasculaire, ou induites par un manque d'irrigation sanguine ;
- les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes ;
- les dégénérescences, héréditaires, traumatiques ou liées au vieillissement des neurones sensoriels de l'ouïe entraînant une diminution ou une perte de l'audition.

Une partie des voies de signalisation affectées dans ces pathologies sont communes à un grand nombre de maladies neurodégénératives. La maladie d'Alzheimer est la démence la plus fréquente. Elle fait apparaître une atrophie du cerveau, une perte neuronale prédominante dans la corne d'Ammon et elle touche aussi les neurones cholinergiques. D'autres pathologies, comme les atrophie lobaires (maladie de Pick, la maladie de Creutzfeld-Jakob), la démence avec corps de Lewy, les démences vasculaires, la maladie de Parkinson sont associées à une mort neuronale importante à l'origine des symptômes de ces démences.

Il n'existe pas actuellement de traitement efficace pour enrayer les dégénérescences neuronales. Une approche thérapeutique pour protéger les neurones de la mort est l'apport de protéines neurotrophiques.

Ces protéines, telles que BDNF (brain-derived neurotrophic factor), CNTF (ciliary neurotrophic factor), NGF (nerve growth factor), GDNF (glia-derived neurotrophic factor) sont synthétisées au cours du développement embryonnaire ou après lésion chez l'adulte. Ces facteurs de croissance favorisent la survie, la maturation et la différentiation des cellules neuronales. De plus, ils inhibent les mécanismes apoptotiques, activent de multiples voies de survie et protègent un grand nombre de populations neuronales. Leur utilisation est proposée dans la plupart des dégénérescences neuronales.

Des composés qui activeraient l'expression de facteurs neurotrophiques ou qui mimeraient l'action de ces facteurs ont un potentiel thérapeutique pour le traitement des syndromes neurodégénératifs.

En particulier, l'apport de molécules neurotrophiques pour le traitement des dégénérescences neuronales vise trois objectifs :
- compenser une carence potentielle en facteurs neurotrophiques liée à un défaut d'apport par les cibles périphériques ou centrales des neurones et/ou un trouble du transport rétrograde de ces facteurs;
- intervenir de façon non spécifique sur des voies biochimiques impliquées dans la cascade dégénérative;
- favoriser les phénomènes compensateurs naturels de croissance dendritique et d'arborisation des terminaisons nerveuses.

Ces composés présenteraient donc un effet bénéfique dans un grand nombre de pathologies en particulier dans les pathologies touchant les systèmes nerveux périphérique et central.

Par ailleurs, dans le cadre ci-dessus, les motoneurones sont des neurones notamment présents dans la moelle épinière et le tronc cérébral. Leur dégénérescence ou leur mort peut conduire à une faiblesse progressive des muscles des membres, puis à une atrophie et éventuellement à une spasticité (c'est à dire une contraction permanente) du muscle.

Les pathologies les plus importantes qui résultent de la dégénérescence et de la mort des motoneurones spinaux et/ou bulbaires sont la sclérose latérale amyotrophique, également connue sous le nom de maladie de Charcot ou encore maladie de Lou Gehrig, et les amyotrophies spinales infantiles, également connues sous les noms de maladie de Werdnig-Hoffmann ou maladie de Kugelberg-Welander.

En outre, on observe une dégénérescence des motoneurones dans les cas de traumatismes avec écrasement et/ou section de la moelle épinière ou des nerfs moteurs périphériques.

Plus généralement, on parle d'amyotrophies spinales pour les maladies où est impliquée la dégénérescence ou la mort des motoneurones de la moelle épinière.

La sclérose latérale amyotrophique (SLA ou ALS pour Amyotrophic Lateral Sclerosis) est une maladie neurodégénérative associée à différents types d'inclusions tels les corps de Lewy et caractérisée par une dégénérescence des motoneurones spinaux et corticaux dont l'issue fatale est parfois associée à une démence frontale. Au cours du développement de l'ALS, les phénomènes dégénératifs se produisent non seulement dans le cerveau mais également dans la moelle épinière et en conséquence dans le muscle, par défaut d'innervation.

On recherche toujours des composés actifs pour lutter contre les affections évoquées ci-dessus.
EP 0 442 350A décrit l'utilisation de stigmasta-4-èn-3-one ou stigmasta- 4-22-dièn-3-one pour le traitement des tumeurs.
HORITA, KIYOSHI et al ("Anti-arrhythmia constituent in Herba leonuri" NATURAL MEDICINES, 56(5), 212-214, 2002), décrit l'activité antiarythmique de stigmast-4-èn-3-one (beta-sitosterone) et l'utilisation médicale des extraits de Herba lenuri le contenant.
YANG, Y. et al ("Anti-emetic principles of Pogostemon cablin" PHYTOMEDICINE , 6(2), 89-93, 1999) décrit l'effet anti-émétique de stigmast-4-èn-3-one et l'utilisation de herbes médicales chinoises le contenant.
KOLAK, UFUK et al ("Cardioactive diterpenoids from the roots of Salvia amplexicaulis" PLANTA MEDICA , 67(8), 761-763, 2001) décrit l'effet vasodépressif de stigmast-4-èn-3-one et l'utilisation médicale des racines de Salvia amplexicaulis le contenant.
SUZUKI K et al ("The cholesterol metabolite cholest-4-en-3-one and its 3-oxo derivatives suppress body weight gain, body fat accumulation and serum lipid concentration in mice" BIOORGANIC & MEDICINAL CHEMISTRY LETTERS, 8(16), 2133-2138,1998) décrit l'effet anti-obésité des dérivés du cholesten-3-one répondant à la formule I).
KANEKO, EMI et al ("Induction of Intestinal ATP-binding Cassette Transporters by a Phytosterol-derived Liver X Receptor Agonist" JOURNAL OF BIOLOGICAL CHEMISTRY , 278(38), 36091-36098, 2003) décrit l'effet agoniste des récepteurs LXRs de ergosta-1,4,6,22-tetraèn-3-one et sa possible utilisation thérapeutique pour la modulation du métabolisme du cholestérol.
LIN, WEI-YU et al ("Anti-platelet aggregation and chemical constituents from the rhizome of Gynura japonica" PLANTA MEDICA , 69(8), 757-764, 2003) décrit l'activité anti-agrégation des plaquettes de stigmasta-1,2,22-trien-3-one et stigmasta-1,4-dien-3-one, et l'utilisation médicale du rhizome de Gynura Japonica les contenant.
La demande de brevet Japonais JP2002030096 le stigmast-22-èn-3-one et son oxime sont divulgué en tant que intermédiaires de synthèse dans la préparation d'ergosténols, ces derniers étant décrits en tant que régénérateurs des projections neuronales avec application dans la thérapie des maladies de Alzheimer et de Parkinson.
UENSEREN E ("Gamma irradiation of cholestenone oximes" INIS ATOMINDEX, INTERNATIONAL ATOMIC ENERGY AGENCY, vol. 8, no. 6, 1977, pages 1-21) divulgue les composé cholest-4-èn-3-one et son oxime en tant que intermédiaires dans la synthèse des azastéroïdes.
Ces documents de l'art antérieure ne décrivent pas une utilisation de composés de l'invention en tant que médicaments neuroprotecteurs.

Or la demanderesse a découvert que des dérivés de la 4-cholestèn-3-one et notamment l'oxime de cholest-4-èn-3-one étaient doués de remarquables propriétés neuroprotectrices, particulièrement vis à vis des motoneurones, des neurones du système nerveux central, des nerfs moteurs et périphériques, et donc étaient utiles comme médicaments et que certains d'entre eux étaient en outre doués de remarquables propriétés inhibitrices de l'effet de modulateurs allostériques positifs des récepteurs GABA_{A} (gamma amino butyrique acide de type A).

C'est pourquoi la présente invention a pour objet les composés répondant à la formule I 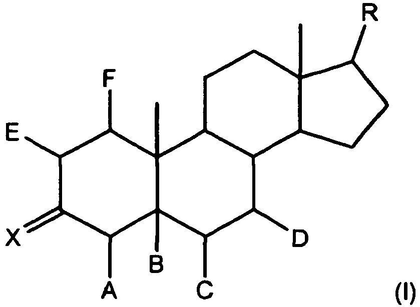 dans laquelle X représente un groupement = N-OH,

R représente un groupement choisi parmi

| | |
|---|---|
| R1 = | 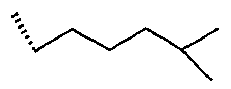 |
| R2 = | 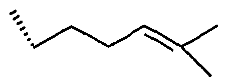 |
| R3 = | 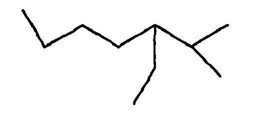 |
| R4 = | 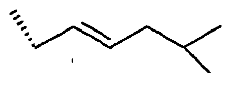 |
| R6 = | 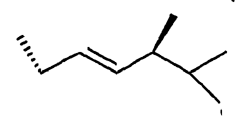 |
| R5= | 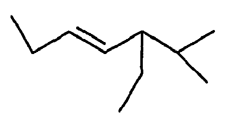 |

A représente un atome d'hydrogène ou ensemble avec B une liaison carbone-carbone,
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone, ainsi que leurs sels d'addition avec les acides pharmaceutiquement acceptables, pour leur utilisation dans une méthode de traitement thérapeutique du corps humain ou animal, c'est-à-dire à titre de médicaments.

Les sels d'addition avec les acides pharmaceutiquement acceptables peuvent être par exemple des sels formés avec les acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

On retient plus particulièrement les composés ci-dessus pour lesquels,
- X représente un groupement = N-OH , A représente ensemble avec B une liaison carbone-carbone, C ,D, représentent un atome d'hydrogène, E,F représentent un atome d'hydrogène ou ensemble une liaison carbone-carbone et R a la signification R1,
- X représente un groupement = N-OH , A représente ensemble avec B une liaison carbone-carbone, C ,D représentent un atome d'hydrogène, E,F représentent un atome d'hydrogène et R a la signification R2 ou R3 ou R4,
- X représente un groupement = N-OH , A représente ensemble avec B une double liaison, C représente ensemble avec D une liaison carbone-carbone, E,F représentent un atome d'hydrogène et R a la signification R1 ou R6,
- X représente un groupement = N-OH , A représente ensemble avec B une double liaison, C représente ensemble avec D une liaison carbone-carbone, E représente ensemble avec F une liaison carbone-carbone et R a la signification R1
- X représente un groupement = N-OH , E représente ensemble avec F une double liaison, C,D,A,B représentent un atome d'hydrogène et R a la signification R1
ainsi que leurs sels d'addition avec les acides pharmaceutiquement acceptables.

Parmi les composés de l'invention on peut citer de plus
- la 5 beta hydroxy cholestan 3 one
- l'oxime de cholestan-3-one
ainsi que leurs sels d'addition avec les acides pharmaceutiquement acceptables.

On retient tout particulièrement
- l'oxime de cholest-4-èn-3-one,
- l'oxime de 1, 4-cholestadièn-3-one
ainsi que leurs sels d'addition avec les acides pharmaceutiquement acceptables.

Les composés objet de la présente invention possèdent de très intéressantes propriétés pharmacologiques. Ils sont doués notamment de remarquables propriétés neuroprotectrices, particulièrement vis à vis des motoneurones.

Ces propriétés sont illustrées ci-après dans la partie expérimentale. Elles justifient l'utilisation des composés ci-dessus décrits ainsi que de leurs sels d'addition avec les acides pharmaceutiquement acceptables à titre de médicament.

Les médicaments selon la présente invention trouvent leur emploi en raison de leurs propriétés neuroprotectrices par exemple dans le traitement ou à la prévention des affections neurodégénératives, comme par exemple la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitements anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, et notamment les amyotrophies spinales, la sclérose latérale amyotrophique et les pathologies dues aux traumatismes de la moelle épinière ou des nerfs moteurs périphériques.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie), etc. Le traitement peut en outre être réalisé en combinaison avec d'autres ingrédients ou traitements, tels que notamment d'autres composés actifs pour traiter les pathologies ou traumatismes spécifiés dans la présente demande.

Ils présentent aussi des propriétés inhibitrices de l'effet de modulateurs allostériques positifs des récepteurs GABA_{A} (gamma amino butyrique acide de type A). Des exemples de modulateurs allostériques positifs sont : l'alloprégnanolone ou 3α-hydroxy-5α-prégnan-20-one ou 3α,5α-TH-PROG ou la tétrahydrodéoxycorticostérone = 3α,20-dihydroxy-5α-pregnan-20-one = 3α,5α-TH-DOC, qui sont des neurostéroïdes endogènes du système nerveux central. Ces propriétés particulières justifient que les composés de l'invention trouvent aussi leur emploi dans le traitement de différentes situations physio-pathologiques où ces neurostéroïdes ont été décrits pour jouer un rôle important, comme par exemple,
- le comportement sexuel
- la mort subite du nouveau-né, et notamment
- la sensibilité douloureuse ou les douleurs neuropathiques chroniques
- l'anxiété ou la dépression
- les lésions traumatiques du système nerveux
- les épilepsies sensibles aux stéroïdes, les troubles du sommeil ou l'intoxication alcoolique
- l'apprentissage cognitif ou les troubles de la mémoire.

Ils trouvent notamment en raison de leurs propriétés neuroprotectrices vis à vis des motoneurones, leur emploi particulièrement dans le traitement des amyotrophies spinales, notamment de la sclérose latérale amyotrophique ou des amyotrophies spinales infantiles, et dans le traitement des traumatismes de la moelle épinière ou des nerfs moteurs périphériques comme évoqué ci-dessus.

En général la dose journalière du composé sera la dose minimum pour obtenir l'effet thérapeutique. Cette dose dépendra des différents facteurs cités auparavant. Les doses des composés de ci-dessus décrits et par exemple de l'oxime de cholest-4-èn-3-one seront en général comprises entre 0,001 à 100 mg par kilo par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.

L'invention a aussi pour objet les compositions pharmaceutiques qui renferment au moins un composé précité ou un de ses sels d'addition avec les acides pharmaceutiquement acceptables, à titre de principe actif.

Dans ces compositions, le principe actif est avantageusement présent à des doses physiologiquement efficaces ; les compositions précitées renferment notamment une dose neuroprotectrice efficace d'au moins un principe actif ci-dessus.

A titre de médicaments, les composés répondant à la formule I ainsi que leurs sels d'addition avec les acides pharmaceutiquement acceptables peuvent être incorporés dans des compositions pharmaceutiques destinées à la voie digestive ou parentérale.

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint d'une pathologie ou d'un traumatisme lié à la dégénérescence ou à la mort des motoneurones tel que défini ci-dessus.

Les compositions pharmaceutiques ou médicaments selon l'invention comprennent avantageusement un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intra-musculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple. Il est entendu que le débit et/ou la dose injectée, ou de manière générale la dose à administrer, peuvent être adaptés par l'homme du métier en fonction du patient, de la pathologie, du mode d'administration, etc.. Il est entendu que des administrations répétées peuvent être réalisées, éventuellement en combinaison avec d'autres ingrédients actifs ou tout véhicule acceptable sur le plan pharmaceutique (tampons, solutions saline, isotonique, en présence d'agents stabilisants, etc.).

L'invention est utilisable chez les mammifères, notamment chez l'être humain.

La présente invention décrit également un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

Les composés de formule I tels que définis ci-dessus sont connus ou peuvent être préparés selon des procédés décrits dans la littérature. Certains dérivés de formule I sont des produits nouveaux.

C'est pourquoi la présente demande a aussi pour objet les composés nouveaux répondant à la formule I 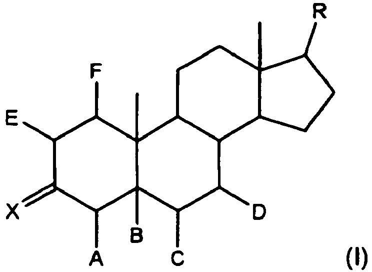 dans laquelle X représente un radical =N-OH et
- A représente ensemble avec B une liaison carbone-carbone, C, D représentent un atome d'hydrogène, E représente ensemble avec F une liaison carbone-carbone et R a la signification R1, ou
- A représente ensemble avec B une liaison carbone-carbone, C ,D représentent un atome d'hydrogène, E, F représentent un atome d'hydrogène et R a la signification R2 ou R4, ou
- A représente ensemble avec B une liaison carbone-carbone, C représente ensemble avec D une liaison carbone-carbone, E, F représentent un atome d'hydrogène et R a la signification R6, ou
- A représente ensemble avec B une liaison carbone-carbone, C représente ensemble avec D une liaison carbone-carbone, E représente ensemble avec F une liaison carbone-carbone et R a la signification R1,
ainsi que leurs sels d'addition avec les acides minéraux ou organiques.

La présente invention décrit également un procédé de préparation des nouveaux composés de formule I tels que définis ci-dessus ainsi que de leurs sels, caractérisé en ce que l'on fait réagir un composé de formule II 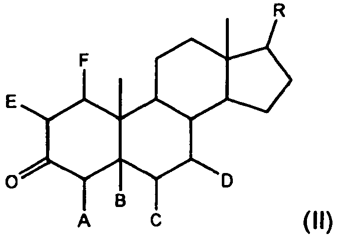 dans laquelle
- A représente ensemble avec B une liaison carbone-carbone, C, D représentent un atome d'hydrogène, E représente ensemble avec F une liaison carbone-carbone et R a la signification R1, ou
- A représente ensemble avec B une liaison carbone-carbone, C ,D représentent un atome d'hydrogène, E, F représentent un atome d'hydrogène et R a la signification R2 ou R4, ou
- A représente ensemble avec B une liaison carbone-carbone, C représente ensemble avec D une liaison carbone-carbone, E, F représentent un atome d'hydrogène et R a la signification R6, ou
- A représente ensemble avec B une liaison carbone-carbone, C représente ensemble avec D une liaison carbone-carbone, E représente ensemble avec F une liaison carbone-carbone et R a la signification R1,
, avec un halogénure d'hydroxylamine comme le chlorhydrate d'hydroxylamine, pour obtenir le composé de formule l'attendu que l'on isole et si désiré salifie.

Dans des conditions préférentielles de mise en oeuvre du procédé ci-dessus décrit,
- on solubilise le produit de départ dans un minimum d'un solvant adapté comme la pyridine
- si une autre fonction cétone est présente, elle est bloquée spécifiquement par un groupement protecteur adapté comme des acétals cycliques,
- on utilise un excès, par exemple 2 équivalents, d'halogénure d'hydroxylamine.
- on opère sous agitation pendant environ 24h à température ambiante.

Les composés de formule II sont des dérivés connus, décrits dans la littérature, et sont accessibles commercialement.

L'invention a encore pour objet l'utilisation d'un composé de formule I 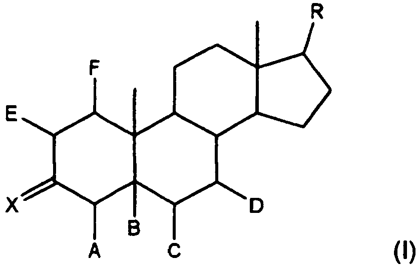 dans laquelle X représente un atome d'oxygène ou un groupement = N-OH, R représente un groupement choisi parmi

| | |
|---|---|
| R1 = | 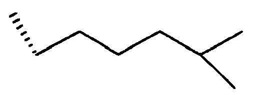 |
| R2 = | 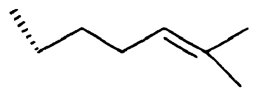 |
| R3 = | 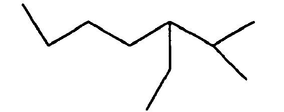 |
| R4 = | 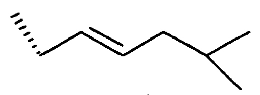 |
| R6 = | 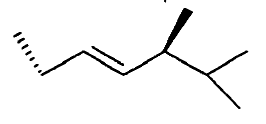 |
| R5= | 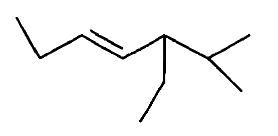 |

A représente un atome d'hydrogène ou ensemble avec B une liaison carbone-carbone
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou de l'un de ses sels d'addition avec les acides pharmaceutiquement acceptables, pour l'obtention d'un médicament neuroprotecteur, notamment destiné au traitement des maladies neurodégénératives comme par exemple la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones et plus particulièrement les amyotrophies spinales particulièrement infantiles, la sclérose latérale amyotrophique, la sclérose en plaques et les traumatismes de la moelle épinière ou des nerfs moteurs périphériques.

L'invention a particulièrement pour objet l'utilisation d'un composé de formule I ci-dessus pour l'obtention d'un médicament neuroprotecteur, notamment destiné au traitement de pathologies ou traumatismes liés à la dégénérescence ou à la mort des neurones, chez des mammifères (en général des patients) atteints de telles pathologies ou traumatismes.

L'invention a plus particulièrement pour objet l'utilisation d'un composé de formule I pour l'obtention d'un médicament destiné au traitement des amyotrophies spinales infantiles et les scléroses latérales amyotrophiques.

L'invention décrit encore l'utilisation d'un composé de formule I ci-dessus pour l'obtention d'un médicament destiné au traitement des pathologies où la suractivation des récepteurs GABA_{A}, (par exemple du fait de la présence de neurostéroïdes comme l'alloprégnanolone et/ou la tétrahydrodéoxycorticostérone), peut avoir un effet néfaste comme les épilepsies sensibles aux stéroïdes, l'intoxication alcoolique, l'apprentissage cognitif, la sensibilité douloureuse ou les troubles du sommeil. Cette suractivation des récepteurs GABA_{A}, peut se caractériser par des effets inhibiteurs ou des effets excitateurs.

La mise en oeuvre de ces médicaments comprend habituellement l'administration à ces mammifères d'une quantité thérapeutiquement efficace d'un composé de formule I et notamment de l'oxime de cholest-4-èn-3-one, en particulier pour augmenter la survie des neurones ou favoriser la croissance axonale.

L'invention a pour objet tout autant la mise à disposition de nouveaux dérivés de la 4-cholestèn-3-one que d'autres dérivés de la 4-cholestèn-3-one que ceux pouvant avoir été décrits dans l'état de la technique. Sont donc exclus ceux décrits dans la littérature.

Les conditions préférentielles ci-dessus décrites de mise en oeuvre des médicaments de formule I s'appliquent également aux autres objets de l'invention visés ci-dessus, notamment aux compositions, nouveaux dérivés, utilisations et inversement.

Les exemples qui suivent illustrent la présente demande.

### EXEMPLE 1

On a préparé une suspension répondant à la formule

| | |
|---|---|
| Oxime de cholest-4-èn-3-one | 20 mg par ml |
| Excipient : | Emulsion huileuse |

### EXEMPLE 2

On a préparé des gélules molles répondant à la formule

| | |
|---|---|
| Oxime de cholest-4-èn-3-one | 250 mg |
| Excipient : quantité suffisante pour une gélule terminée à | 750 mg |

### EXEMPLE 3 : Oxime de 1, 4-cholestadièn-3-one (R=R1)

Dans un ballon de 100 ml, on solubilise 5 g de 1, 4-cholestadièn-3-one (13 mmol) dans 50 ml de pyridine, puis on ajoute 5 g de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction. La phase organique est lavée avec une solution aqueuse acidifiée (HCl 1 %). L'acétate d'éthyle est évaporé sous pression réduite. On obtient une poudre blanche avec un rendement supérieur à 50%.

### Analyse

- Chromatographie Liquide / Spectrométrie de Masse (Electrospray®)
- Conditions de la chromatographie liquide haute performance :
   Colonne : Macherey-Nagel - Nucleosil® 300-6 C4 - 150x4,6 mm
   Gradient : eau (+0.05% TFA) / acétonitrile (+0,05% acide trifluoroacétique)
      t= 0 min : 60% acétonitrile, 40% H₂O
      t= 6 min : 100% acétonitrile, 0% H₂O
      Puis on reste 5 min à 100% d'acétonitrile
   Temps de rétention : 5 min 60 secondes
   Pic détecté en spectrométrie de masse : {M+H}⁺ = 398

### EXEMPLE 4 : Oxime de 4, 24-cholestadièn-3-one (R = R 2)

Dans un ballon de 10 ml, on solubilise 100 mg de 4, 24-cholestadièn-3-one (0,26 mmol) dans 5 ml de pyridine, puis on ajoute 100 mg de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction. Puis la phase organique est lavée avec une solution aqueuse acidifiée (HCl 1%). L'acétate d'éthyle est évaporé sous pression réduite. On obtient une poudre blanche avec un rendement supérieur à 50%.

### Analyse

- Chromatographie Liquide / Spectrométrie de Masse (Electrospray®) Pic détecté en spectrométrie de masse : {M+H}⁺=398

### EXEMPLE 5 : Oxime de 4, 22-cholesiadièn-3-one (R=R4)

Dans un ballon de 10 ml, on solubilise 10 mg de 4, 22-cholestadièn-3-one (0,026 mmol) dans 5 ml de pyridine, puis on ajoute 100 mg de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction. Puis la phase organique est lavée avec une solution aqueuse acidifiée (HCl 1%). L'acétate d'éthyle est évaporé sous pression réduite. On obtient une poudre blanche avec un rendement supérieur à 50%.

### Analyse

- Chromatographie Liquide / Spectrométrie de Masse (Electrospray®)
   Pic détecté en spectrométrie de masse : {M+H}⁺ =398

### EXEMPLE 6 : Oxime de 4-stigmasta-ène-3-one, (R = R 3)

Dans un ballon de 50 ml, on solubilise 100 mg de 4-stigmasta-ène-3-one (0,24 mmol) dans 10 ml de pyridine, puis on ajoute 100 mg de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction. Puis la phase organique est lavée avec une solution aqueuse acidifiée (HCl 1%). L'acétate d'éthyle est évaporé sous pression réduite. On obtient une poudre blanche avec un rendement supérieur à 50%.

### Analyse

- Chromatographie Liquidé / Spectrométrie de Masse (Electrospray®)
- Pic détecté en spectrométrie de masse : {M+H}⁺=428

### EXEMPLE 7 : Oxime de 4,6, 22-Ergosta-trién-3-one (R = R 6)

Dans un ballon de 50 ml, on solubilise 100 mg de 4,6, 22-Ergosta-trièn-3-one (0,25 mmol) dans 10 ml de pyridine, puis on ajoute 100 mg de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction. Puis la phase organique est lavée avec une solution aqueuse acidifiée (HCl 1%). L'acétate d'éthyle est évaporé sous pression réduite. On obtient une poudre blanche avec un rendement supérieur à 50%.

### Analyse

- Chromatographie Liquide / Spectrométrie de Masse (Electrospray®)
- Pic détecté en spectrométrie de masse : {M+H}⁺=410

### EXEMPLE 8 : Oxime de 1,4, 6-Cholesta-trièn-3-one (R = R 1)

Dans un ballon de 50 ml, on solubilise 100 mg de 1,4, 6-Cholesta-trièn-3-one (0,26 mmol) dans 10 ml de pyridine, puis on ajoute 100 mg de chlorhydrate d'hydroxylamine. L'agitation est maintenue pendant 24h à température ambiante, et on évapore le solvant sous pression réduite. On ajoute de l'eau, puis de l'acétate d'éthyle afin d'effectuer une extraction, puis la phase organique est lavée avec une solution aqueuse acidifiée (HCl 1%). L'acétate d'éthyle est évaporé sous pression réduite. On obtient une poudre blanche avec un rendement supérieur à 50%.

### Analyse

- Chromatographie Liquide / Spectrométrie de Masse (Electrospray®)
- Pic détecté en spectrométrie de masse : {M+H}⁺ =396

### ETUDE PHARMACOLOGIQUE

On a testé les composés de formule 1 suivants:

| Composé N° | |
|---|---|
| 1 | Oxime de cholest-4-èn-3-one décrite dans Gamma irradiation of cholestenone oximes. Uenseren, Envare. Cekmece Nucl. Res. Train. Cent., Istanbul, Turk. Avail. INIS. Report (1976), (CNAEM-R-157), 21 pp. From: INIS Atomindex 1977, 8(6), Abstr. No. 295540. |
| 2 | Composé de l'exemple 3 |
| 3 | Oxime de cholestan-3-one disponible commercialement chez Sigma-Aldrich |
| 4 | Cholest-4,24-dien-3-one décrite dans Chemical synthesis of cholest-5,7,24-trien-3béta-ol and demonstration of its conversion to cholesterol in the rat. Scallen, Terence J. Univ. of Minnesota, Minneapolis, Biochemical and Biophysical Research Communications (1965), 21(2), 149-55. |
| 5 | 4,22-cholest-dièn-3-one. décrit dans 3-Keto-Δ4-steroids from 3-hydroxy-ène-4 (or ène-5)-steroids. Yamanaka, Toru; Imai, Takashi. (Takasago Perfumery Co., Ltd., Japan). JP 52116456 19770929 Showa.. Application: JP 76-32979 19760325. CAN 88:136852 AN 1978:136852 |
| 6 | Composé de l'exemple 4 |
| 7 | Composé de l'exemple 5 |
| 8 | Composé de l'exemple 6 |
| 9 | 5-béta-hydroxy-cholestan-3-one disponible commercialement chez Sigma-Aldrich |
| 10 | 5-alpha-hydroxy-cholestan-3-one disponible commercialement chez Sigma-Aldrich |
| 11 | 5-alpha-cholest-6-èn-3-one disponible commercialement chez Sigma-Aldrich |
| 12 | 24-méthylcholest-4,6,22-trièn-3-one décrite dans Preparation of 3-béta-hydroxy-(24R)-methylcholest-5-ene. Khripach, V. A.; Zhabinskii, V. N.; Zhemosek, E. V.; Lakhvich, F. A. (Institute of Bioorganic Chemistry, Academy of Sciences, Belorussian S.S.R., USSR). U.S.S.R. (1990), et SU 1594181 19900923 : SU 88-4612746 19881201. CAN 114:102558 AN 1991:102558 |
| 13 | Oxime de cholest-5-èn-3-one-décrit dans Gamma irradiation of cholestenone oximes. Uenseren, Envare. Cekmece Nucl. Res. Train. Cent. Istanbul, Turk. Avail. INIS. Report (1976), (CNAEM-R-157), 21 pp. |
| 14 | Oxime de cholest-4,6-dièn-3-one décrit dans Azasteroid from cholesta-4,6-dien-3-one. Ahmad, Mohammed S.; Siddiqui, A. H.; Shafiullah; Logani, S. C. Aligarh Muslim Univ., Aligarh, India. Australian Journal of Chemistry (1969), 22(1), 271-4. |
| 15 | Composé de l'exemple 7 |
| 16 | Oxime de 5- alpha-cholest-1-èn-3-one décrite dans Polyphosphoric acid-catalyzed Beckmann rearrangement of 3-keto-steroid oximes. Kobayashi, Masaru; Shimizu, Yuzuru; Mitsuhashi, Hiroshi. Fac. Pharm. Sci., Hokkaido Univ., Sapporo, Japan. Chemical & Pharmaceutical Bulletin (1969), 17(6), 1255-60. |
| 17 | Composé de l'exemple 8 |

### 1. Effets des composés de formule I sur la survie des motoneurones

Pour mettre en évidence l'action neuroprotectrice des composés de formule I, la demanderesse a étudié leur activité sur un modèle *in vitro* de privation trophique de motoneurones de rats. On pourra se référer utilement à la demande de brevet WO 0142784 de la demanderesse sur la mise en culture des motoneurones de moelle épinière.

La moelle épinière d'embryons E14 de rat est disséquée et la partie ventrale est dissociée par trituration après trypsination. Les motoneurones sont séparés des autres cellules spinales par une méthode connue (Camu et al, 1993, Purification of spinal motoneurons from chicken and rat embryos by immunopanning. In « Immunoselection Strategies for Neural cell culture », Neuroprotocols : A companion to Methods in Neurosciences 2, 191-199 ; Henderson et al., 1993, Neutrophins promote motor neuron survival and are present in embryonic limb bud. Nature 363 (6426) :266-70). Les cellules sont centrifugées sur un gradient de densité. Les motoneurones sont enrichis dans la fraction des grandes cellules (les moins denses). Les cellules de cette fraction sont incubées avec un anticorps anti-p75, un antigène de surface présent sur les motoneurones. Des anticorps secondaires couplés à des billes magnétiques sont ajoutés et le mélange de cellules est passé à travers une colonne dans un aimant (Arce et al, 1999). Seuls les motoneurones sont retenus : leur pureté est de l'ordre de 90%.

Les motoneurones sont ensemencés à faible densité dans des puits de culture sur un substrat de polyornithine-laminine dans un milieu neurobasal supplémenté selon Raoul et al, 1999, Programmed cell death of embryonic motoneurons triggered through the Fas death receptor. J Cell Biol 147(5) :1049-62. Des contrôles négatifs (absence de facteurs trophiques) et positifs (en présence de BDNF (Brain-Derived Neurotrophic Factor) à 1 ng/ml, GDNF (Glial-Derived Neurotrophic Factor) à 1 ng/ml et CNTF (Ciliary Neurotrophic Factor) à 10 ng/ml, commercialisés par la société américaine PEPROTECH, Inc. et la société Sigma-Aldrich, sont inclus dans chaque série.

Les composés à tester sont ajoutés 60 minutes après l'ensemencement et les cultures sont maintenues à 37°C sous 5% de CO2 pendant 3 jours.

Les motoneurones ont une tendance spontanée à mourir en l'absence de facteurs neurotrophiques (Pettmann et Henderson, 1998, Neuronal cell death. Neuron 20(4) :633-47). Après 3 jours, la survie est évaluée par une mesure de fluorescence après incubation des cellules en présence de calcéine qui devient fluorescente dans les cellules vivantes.

Après 3 jours en culture à 37°C, sous 5% de CO2 et en humidité saturante, jusqu'à 50% des motoneurones ensemencés initialement survivent dans le milieu supplémenté en facteurs neurotrophiques, alors que moins de 15% des motoneurones survivent en milieu basal seul.

L'activité des composés à tester a été évaluée par leur capacité à empêcher la mort des motoneurones quand ils sont additionnés au milieu neurobasal en comparaison avec la survie des motoneurones en milieu supplémenté avec des facteurs neurotrophiques.

Les composés de formule I selon l'invention ont montré une activité à une concentration capable de permettre un meilleurs taux de survie des motoneurones dans le milieu basal. Ce taux de survie est exprimé par un chiffre, le ratio. Si le ratio est supérieur à O, l'effet des composés est positif sur la survie des motoneurones.

Les résultats obtenus sont les suivants :

| Composé n° | Concentration en µM | Ratio |
|---|---|---|
| 1 | 5 | 1 |
| 2 | 5 | 0,6 |
| 6 | 5 | 0,5 |
| 7 | 6 | 0,25 |
| 8 | 10 | 0,3 |
| 15 | 5 | 0,2 |
| 17 | 5 | 0,3 |
| 9 | 1 | 0,5 |

De par leur effet trophique sur les motoneurones spinaux, les composés de formule I selon l'invention se montrent donc utiles comme médicament, notamment dans le traitement des amyotrophies, en particulier dans le traitement de la sclérose latérale amyotrophique ou des amyotrophies spinales infantiles, et dans le traitement des traumatismes de la moelle épinière.

### 2. Effets des composés de formule I sur la neuroprotection

Une axotomie du nerf facial est pratiquée sur des ratons nouveaux-nés âgés de 2-3 jours. Les animaux reçoivent l'oxime de cholest-4-èn-3-one 4 heures avant la section unilatérale du nerf puis quotidiennement pendant 5 jours par voie sous-cutanée. Sept jours après la section du nerf, les animaux sont anesthésiés, puis fixés par perfusion intra-cardiaque de paraformaldéhyde. Le cerveau est alors prélevé, inclus en paraffine. L'analyse histologique de coupes sériées de 7µm du noyau facial, colorées au cresyl violet, permet de compter le nombre de motoneurones du côté intact ainsi que du côté du nerf sectionné (Casanovas et al., Prevention by lamotrigine, MK-801 and N omega-nitro-L-arginine methyl ester of motoneuron cell death after neonatal axotomy, Neuroscience, 1996, 71, 313-325).

Les résultats obtenus sont les suivants :

La survie des motoneurones du noyau facial chez des rats nouveaux-nés axotomisés et traités par l'oxime de cholest-4-èn-3-one est augmentée jusqu'à 40 % à une dose comprise entre 3 et 100 mg/kg, selon la voie d'administration, par comparaison au nerf non sectionné.

### 3. Effets des composés de formule I sur la neuroprotection

Un écrasement du nerf sciatique est pratiqué sur des souris adultes selon la méthode décrite par Azzouz et al.,Enhancement of mouse sciatic nerve regeneration by the long chain fatty alcohol,N-hexacosanol, Exp. Neurol., 1996, 138 :189-97). Les animaux reçoivent les composés à tester le jour de l'écrasement puis chaque jour pendant 4 semaines et par voie sous-cutanée. Chaque semaine les animaux sont anesthésiés pour réaliser un enregistrement électromyographique dans le muscle du mollet après stimulation supramaximale du nerf sciatique. L'amplitude, la durée et la latence des potentiels d'action évoqués (CMAP) sont ainsi mesurées. Quatre semaines après l'écrasement du nerf, les animaux sont sacrifiés par injection d'une dose mortelle d'anesthésique et un segment de nerf lésé est prélevé, fixé en glutaraldéhyde 4 % et inclus en résine. L'analyse histologique de coupes sériées, colorées au bleu de toluidine, permet un comptage semi-automatisé des fibres dégénérées et non-dégénérées, ainsi qu'une mesure de la taille des axones et de l'épaisseur de la gaine de myéline des fibres non-dégénérées.

Les résultats obtenus sont les suivants :

L'administration de l'oxime de cholest-4-èn-3-one réduit de 20 à 40 % le nombre de fibres dégénérées par comparaison aux animaux non traités. De manière encore plus spectaculaire, la régénération des fibres nerveuses est stimulée fortement par les composés à tester dès la deuxième semaine après l'écrasement, avec un effet maximum observé quatre semaines après l'écrasement. Ainsi quatre semaines après l'écrasement, l'amplitude des CMAP chez les souris traitées par les composés à tester à une dose entre 0.3 et 30 mg/kg/jour, selon la voie d'administration, est augmentée de 40 à 70 % et la vitesse de conduction nerveuse améliorée de 30 à 50 % par comparaison aux souris non traitées.

### 4. Effets des composés de formule I sur la protection des neurones striataux de la mort induite par la surexpression d'une forme mutée de la huntingtine

Des cultures primaires de neurones striataux sont préparées comme décrit dans la littérature (Primary striatal neuronal culture, Mao L. et al., Methods Mol Med., 2003, 79:379-86). Les cellules sont électroporées d'après la procédure décrite par Raoul et al., (Motoneuron death triggered by a specific pathway downstream of Fas. potentiation by ALS-linked SOD1 mutations Neuron, 2002, 35:1067-83) avant l'ensemencement avec un vecteur ou plasmide d'expression contenant un élément promoteur suivi de l'ADN codant pour une forme tronquée de la huntingtine qui comprend les 480 premiers amino acides et 68 CAG (Saudou et al., Huntingtin acts in the nucleus to induce apoptosis but death does not correlate with the formation of intranuclear inclusions, Cell, 1998, 95:55-66). Un second vecteur d'expression contenant l'ADN codant la *green fluorescent protein* (GFP) est également électroporé et sert de gène rapporteur. L'ADN du plasmide codant la huntingtine a été préparé par purification au chlorure de césium. Le plasmide contenant la séquence GFP a été préparée sur colonnes Qiagen. L'intégrité des séquences d'ADN est vérifiée par séquençage, transfection et *western blotting.* Les cellules qui survivent à l'électroporation sont ensemencées à une densité de 4000 cellules par puits de plaques 96 puits. La culture se fait dans du milieu Neurobasal (GIBCO) complémenté avec du pyruvate et du B-27 (Beckton Dickinson). Les cellules sont maintenues en culture pendant 7 jours sans changer le milieu.

Les traitements avec les composés à tester se font juste après l'ensemencement à une concentration finale de 1 µM dans 0.5% de diméthylsulfoxyde (DMSO). Les contrôles positifs se font par adjonction de BDNF à 5 ng/ml final. Les contrôles négatifs ne reçoivent que 0.5% de DMSO.

La mort cellulaire est évaluée après les 7 jours par comptage du nombre de cellules vivantes exprimant la GFP.

L'activité des composés à tester a été évaluée par leur capacité à empêcher la mort des neurones striataux cultivés dans le milieu neurobasal en comparaison avec la survie des neurones striataux en milieu supplémenté avec du BDNF (Brain-Derived Neurotrophic Factor).

Les résultats obtenus sont les suivants :

| Composé n° | Concentrations en µM | Ratio |
|---|---|---|
| 1 | 1 | 0,3 |
| 4 | 1 | 0,2 |
| 9 | 1 | 0,5 |
| 11 | 1 | 0,3 |
| 12 | 1 | 0,3 |

A la concentration de 10⁻⁶ M, les composés à tester montrent un effet protecteur de la mort cellulaire induite par la huntingtine mutée jusqu'à 60 % par comparaison aux cellules traitées par le BDNF.

De par leur effet neuroprotecteur, les composés de formule I selon l'invention se montrent donc utiles comme médicaments destiné au traitement ou à la prévention des affections neurodégénératives, notamment dans le traitement des amyotrophies spinale, de la sclérose latérale amyotrophique, dans le traitement des traumatismes de la moelle épinière et des nerfs périphériques et dans le traitement de la maladie de Huntington.

### 5. Effets des composés de formule I sur la potentialisation des récepteurs GABA_{A} par les stéroïdes

Des expériences électrophysiologiques ont été réalisées in vitro avec des neurones de rat (tranches d'hippocampe, neurones sensitifs en culture), comme suit :

Les expériences sont réalisées avec des hippocampes prélevés sur des rats de souche Sprague Dawley âgés de 10 à 21 jours. Les animaux sont sacrifiés par décapitation, et leur cerveau est rapidement transféré dans de l'ACSF (Artificial Cerebro-Spinal Fluid) sans sodium (composition : KCI 2 mM, NaH₂PO₄ 1.2 mM, MgCl₂ 2 mM, CaCl₂ 0.5 mM, NaHCO₃ 26 mM, glucose 11 mM, saccharose 250 mM), maintenu à une température de 4°C et bullé avec du carbogène (mélange 95% O₂, 5% CO₂). Les hippocampes sont extraits de chaque hémisphère cérébral puis débités en tranches de 300 µM d'épaisseur par un « tissue chopper ». Les tranches sont mises à reposer dans du milieu ACSF (composition : NaCl 126 mM, KCI 2 mM, NaH₂PO₄ 1.2 mM, MgCl₂ 2 mM, CaCl₂ 0.5 mM, NaHCO₃ 26 mM, glucose 11 mM) à température ambiante, au moins une heure avant enregistrement. Une tranche est ensuite déposée à l'aide d'une pipette sur un système d'enregistrement multi-électrode (MEA, MultiChannel Systems™, Germany) et positionnée de façon à couvrir les 64 électrodes. Pour éviter toute modification de positionnement par le flux de perfusion, une grille lestée par un « U » en platine est positionnée sur la tranche. Le MEA est alors rapidement inséré dans le système d'enregistrement et la chambre perfusée avec de l'ACSF bullé au carbogène; le milieu de la chambre est relié à la terre. L'ACSF perfusé ainsi que le MEA sont maintenus à une température de 37°C. Une des 60 électrodes est sélectionnée comme électrode de stimulation. Un courant de stimulation biphasique et d'une intensité de 300 µA durant 0,1 ms est généré à partir de cette électrode à raison d'une stimulation toutes les 30 secondes. Les réponses évoquées sont visualisées simultanément au niveau des 59 autres électrodes du MEA. Les axones des collatérales de Schaeffer sont stimulés en CA3. Dans la région CA1, 6 à 12 électrodes sont sélectionnées pour enregistrer les potentiels de champ qui correspondent à la somme des variations des potentiels postsynaptiques des neurones situés au voisinage de chaque électrode. Les molécules testées sont dissoutes dans le milieu ACSF (la concentration finale en DMSO est ≤ 0,1 %) et amenées sur la tranche grâce au système de perfusion.

Les neurones sensitifs de rat sont cultivés 1 à 2 jours et ensuite enregistrés avec la technique de patch-clamp comme décrit dans De Roo M. et al. (Dehydroepiandrosterone potentiates native ionotropic ATP receptors containing the P2X2 subunit in rat sensory neurones, J Physiol, 2003, 552:59-71).

Les résultats obtenus sont les suivants :

Sur les tranches d'hippocampe, une petite fraction des potentiels post-synaptiques de champ enregistrés dans la région CA1 correspond à une transmission GABA_{A} excitatrice: elle est bloquée par des antagonistes classiques des récepteurs GABA_{A} (20 µM bicuculline ou 50 µM picrotoxine) et potentialisée par des modulateurs allostériques positifs des récepteurs GABA_{A}: benzodiazépine (1-10 µM de Diazepam) et stéroïdes (10-1000 nM alloprégnanolone ou tétrahydrodéoxycorticostérone). L'effet potentialisateur des stéroïdes disparaît lorsque les récepteurs GABA_{A} sont désensibilisés par l'ajout d'une concentration saturante de GABA (10 mM). L'effet excitateur du GABA est lié au passage des ions hydrogénocarbonates (HCO3⁻) à travers les récepteurs GABA_{A}.

Dans les neurones sensitifs enregistrés en patch-clamp, des courants sont évoqués en ouvrant sélectivement les récepteurs GABA_{A} de ces neurones par application rapide d'un agoniste GABA_{A}-sélectif, l'isoguvacine. Les courants chlore ainsi évoqués sont bloqués par la bucuculline et potentialisés par l'alloprégnanolone.

Les résultats montrent que l'oxime de cholest-4-èn-3-one (testé seul jusqu'à 10 µM) n'a pas d'effet sur les récepteurs GABA_{A} (tranche d'hippocampe ou neurones sensitifs). Par contre, l'oxime de cholest-4-èn-3-one est capable de bloquer l'effet de modulateurs allostériques positifs (alloprégnanolone ou tétrahydrodéoxycorticostérone) des récepteurs GABA_{A} et ceci de manière dose-dépendante et pour des concentrations équivalentes à celles où sont observés les effets neurotrophiques in vitro.

Les composés de formule I ont donc un effet "protecteur" dans toutes les pathologies où la suractivation des récepteurs GABA_{A}, du fait de la présence d'alloprégnanolone et/ou de tétrahydrodéoxycorticostérone, peut avoir un effet néfaste, que ce soit pour des effets inhibiteurs ou sur des effets excitateurs médiés par les récepteurs GABA_{A}.

### Etude toxicologique

L'administration chez la souris, en particulier de l'oxime de la cholest-4-èn-3-one, par les voies orale, sous-cutanée, intra-péritonéale et intra-veineuse, à des doses allant jusqu'à 300 mg/kg/jour, par traitement avec administration quotidienne pouvant aller jusqu'à 28 jours, n 'a pas montré de toxicité significative.

Chez le singe, l'administration par voie orale de doses croissantes journalières jusqu'à 1500mg/kg sur une période de 10 jours n'a révélé aucune toxicité.

## Revendications

1. Un composé répondant à la formule I 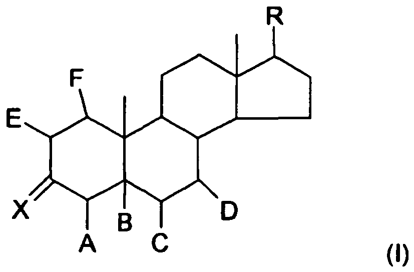 dans laquelle X représente un groupement = N-OH,
R représente un groupement choisi parmi
| | |
|---|---|
| **R1 =** | 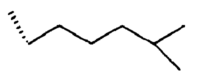 |
| **R2 =** | 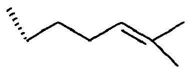 |
| **R3 =** | 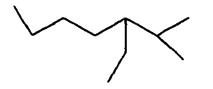 |
| **R4 =** | 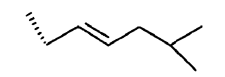 |
| **R6 =** | 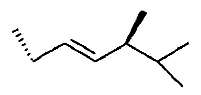 |
| **R5=** | 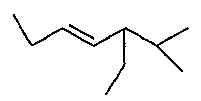 |
A représente un atome d'hydrogène ou ensemble avec B une liaison carbone-carbone,
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses sels d'addition avec les acides pharmaceutiquement acceptables, pour son utilisation dans une méthode de traitement thérapeutique du corps humain ou animal, c'est-à-dire à titre de médicament.

2. Un composé selon la revendication 1 pour son utilisation selon la revendication 1 **caractérisée en ce que** dans la formule I, A représente ensemble avec B une liaison carbone-carbone, C, D, représentent un atome d'hydrogène, E, F représentent un atome d'hydrogène ou ensemble une liaison carbone-carbone et R a la signification R1, c'est-à-dire l'oxime de cholest-4-èn-3-one ou l'oxime de 1, 4-cholestadièn-3-one, ou l'un de ses sels d'addition avec les acides pharmaceutiquement acceptables, pour son utilisation à titre de médicament.

3. Un composé selon la revendication 1 pour son utilisation selon la revendication 1 **caractérisé en ce que** dans la formule I, A représente ensemble avec B une liaison carbone-carbone, C, D représentent un atome d'hydrogène, E, F représentent un atome d'hydrogène et R a la signification R2 ou R3 ou R4, ou l'un de ses sels d'addition avec les acides pharmaceutiquement acceptables, pour son utilisation à titre de médicament.

4. Un composé selon la revendication 1 pour son utilisation selon la revendication 1 **caractérisé en ce que** dans la formule I, A représente ensemble avec B une double liaison, C représente ensemble avec D une liaison carbone-carbone, E, F représentent un atome d'hydrogène et R a la signification R1 ou R6, ou l'un de ses sels d'addition avec les acides pharmaceutiquement acceptables, pour son utilisation à titre de médicament.

5. Un composé selon la revendication 1 pour son utilisation selon la revendication 1 **caractérisé en ce que** dans la formule I A représente ensemble avec B une liaison carbone-carbone, C représente ensemble avec D une liaison carbone-carbone, E représente ensemble avec F une liaison carbone-carbone et R a la signification R1, ou l'un de ses sels d'addition avec les acides pharmaceutiquement acceptables, pour son utilisation à titre de médicament.

6. Un composé selon la revendication 1 pour son utilisation selon la revendication 1 **caractérisé en ce que** dans la formule I E représente ensemble avec F une liaison carbone-carbone, C, D, A, B, représentent un atome d'hydrogène et R a la signification R1, ou l'un de ses sels d'addition avec les acides pharmaceutiquement acceptables, pour son utilisation à titre de médicament.

7. Utilisation d'un composé de formule I 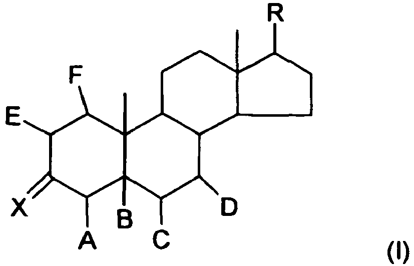 dans laquelle X représente un groupement = N-OH ou un atome d'oxygène,
et R représente un groupement choisi parmi
| | |
|---|---|
| **R1 =** | 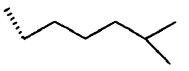 |
| **R2 =** | 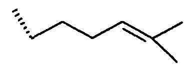 |
| **R3 =** | 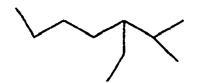 |
| **R4 =** | 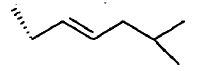 |
| **R6 =** | 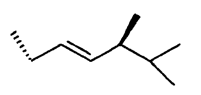 |
| **R5=** | 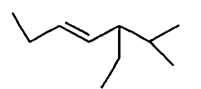 |
A représente un atome d'hydrogène ou ensemble avec B une liaison carbone-carbone,
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses sels d'addition avec les acides pharmaceutiquement acceptables, pour l'obtention d'un médicament destiné à être utilisé comme neuroprotecteur.

8. Utilisation, selon la revendication 7 **caractérisée en ce que** le médicament neuroprotecteur est destiné au traitement d'une maladie neurodégénérative.

9. Utilisation, selon la revendication 7 ou 8 **caractérisée en ce que** le médicament neuroprotecteur est destiné au traitement d'une maladie neurodégénérative choisie parmi la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones les amyotrophies spinales, la sclérose en plaques et les traumatismes de la moelle épinière ou des nerfs moteurs périphériques.

10. Utilisation selon l'une des revendications 7 à 9 **caractérisée en ce que** le médicament neuroprotecteur est destiné au traitement d'une maladie neurodégénérative choisie parmi les pathologies ou traumatismes liés à la dégénérescence ou à la mort des neurones, chez des mammifères atteints de telles pathologies ou traumatismes.

11. Utilisation selon l'une des revendications 7 à 10 **caractérisée en ce que** le médicament neuroprotecteur est destiné au traitement des amyotrophies spinales infantiles.

12. Utilisation selon l'une des revendications 7 à 10 **caractérisée en ce que** le médicament neuroprotecteur est destiné au traitement des scléroses latérales amyotrophiques.

13. Utilisation, selon la revendication 7 **caractérisée en ce que** le médicament neuroprotecteur est destiné au traitement de la sensibilité douloureuse ou des douleurs neuropathiques chroniques.

14. Utilisation, selon la revendication 7 **caractérisée en ce que** le médicament neuroprotecteur est destiné au traitement des lésions traumatiques du système nerveux.

15. Utilisation, selon la revendication 7 **caractérisée en ce que** le médicament neuroprotecteur est destiné au traitement des épilepsies sensibles aux stéroïdes, les troubles du sommeil ou l'intoxication alcoolique.

16. Utilisation, selon la revendication 7 **caractérisée en ce que** le médicament neuroprotecteur est destiné au traitement de l'apprentissage cognitif ou les troubles de la mémoire.

17. Utilisation, selon la revendication 7 **caractérisée en ce que** le médicament neuroprotecteur est destiné au traitement de l'anxiété ou de la dépression.

18. Utilisation selon l'une des revendications 7 à 17 **caractérisée en ce que** le composé de formule I selon la revendication 1 est l'oxime de cholest-4-èn-3-one.

19. Un composé répondant à la formule I 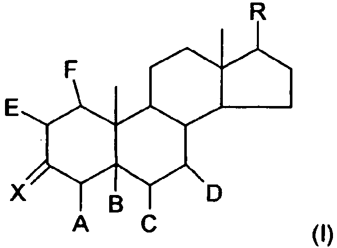 dans laquelle X représente un radical =N-OH et
- A représente ensemble avec B une liaison carbone-carbone, C, D représentent un atome d'hydrogène, E représente ensemble avec F une liaison carbone-carbone et R a la signification R1, ou
- A représente ensemble avec B une liaison carbone-carbone, C, D représentent un atome d'hydrogène, E, F représentent un atome d'hydrogène et R a la signification R2 ou R4, ou
- A représente ensemble avec B une liaison carbone-carbone, C représente ensemble avec D une liaison carbone-carbone, E, F représentent un atome d'hydrogène et R a la signification R6, ou
- A représente ensemble avec B une liaison carbone-carbone, C représente ensemble avec D une liaison carbone-carbone, E représente ensemble avec F une liaison carbone-carbone et R a la signification R1,
ainsi que ses sels d'addition avec les acides minéraux ou organiques.

20. Une composition pharmaceutique **caractérisée en ce qu'**elle renferme à titre de principe actif l'un au moins des médicaments tels que définis à la revendication 1, ainsi qu'un excipient pharmaceutiquement acceptable.

21. Une composition pharmaceutique **caractérisée en ce qu'**elle renferme à titre de principe actif l'un au moins des médicaments tels que définis à la revendication 2 ainsi qu'un excipient pharmaceutiquement acceptable.

22. Une composition pharmaceutique **caractérisée en ce qu'**elle renferme à titre de principe actif l'un au moins des médicaments tels que définis à l'une quelconque des revendications 3 à 6, ainsi qu'un excipient pharmaceutiquement acceptable.

23. Une composition pharmaceutique selon l'une quelconque des revendications 21 à 22, **caractérisée en ce que** l'excipient pharmaceutiquement acceptable est au moins une huile végétale ou animale.

## Claims

1. A compound corresponding to formula I 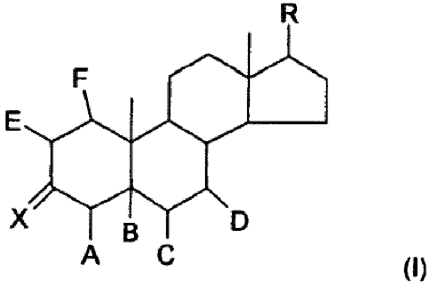 in which X represents an = N-OH group,
R represents a group chosen from
| | |
|---|---|
| **R1 =** | 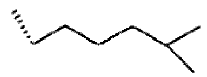 |
| **R2 =** | 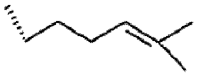 |
| **R3 =** | 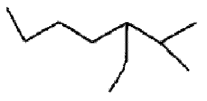 |
| | 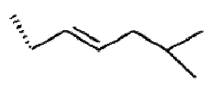 |
| **R6 =** | 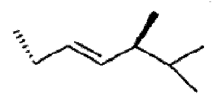 |
| **R5=** | 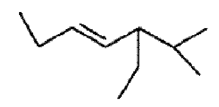 |
A represents a hydrogen atom or together with B a carbon-carbon bond,
B represents a hydrogen atom, a hydroxy group or together with A a carbon-carbon bond,
C represents a hydrogen atom or together with D a carbon-carbon bond,
D represents a hydrogen atom or together with C a carbon-carbon bond,
E represents a hydrogen atom or together with F a carbon-carbon bond,
F represents a hydrogen atom or together with E a carbon-carbon bond, or one of its addition salts with pharmaceutically acceptable acids, for use in a therapeutic treatment method for the human or animal body, i.e. as a medicament.

2. A compound according to claim 1 for use according to claim 1, **characterized in that** in formula I, A together with B represents a carbon-carbon bond, C, D, represent a hydrogen atom, E, F represent a hydrogen atom or together a carbon-carbon bond, and R has the meaning R1, i.e. the oxime of cholest-4-en-3-one or the oxime of 1,4-cholestadien-3-one, or one of its addition salts with pharmaceutically acceptable acids, for use as a medicament.

3. A compound according to claim 1 for use according to claim 1, **characterized in that** in formula I, A together with B represents a carbon-carbon bond, C, D represent a hydrogen atom, E, F represent a hydrogen atom and R has the meaning R2 or R3 or R4, or one of its addition salts with pharmaceutically acceptable acids, for use as a medicament.

4. A compound according to claim 1 for use according to claim 1, **characterized in that** in formula I, A together with represents B a double bond, C together with D represents a carbon-carbon bond, E, F represent a hydrogen atom and R has the meaning R1 or R6, or one of its addition salts with pharmaceutically acceptable acids, for use as a medicament.

5. A compound according to claim 1 for use according to claim 1, **characterized in that** in formula I A together with B represents a carbon-carbon bond, C together with D represents a carbon-carbon bond, E together with F represents a carbon-carbon bond and R has the meaning R1, or one of its addition salts with pharmaceutically acceptable acids, for use as a medicament.

6. A compound according to claim 1 for use according to claim 1, **characterized in that** in formula I E together with F represents a carbon-carbon bond, C, D, A, B, represent a hydrogen atom and R has the meaning R1, or one of its addition salts with pharmaceutically acceptable acids, for use as a medicament.

7. Use of a compound of formula I 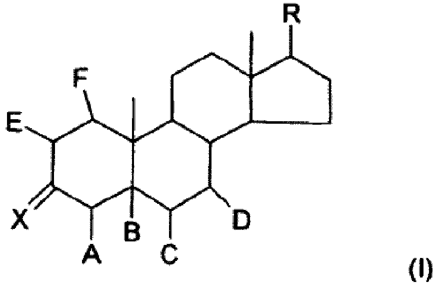 in which X represents an = N-OH group or an oxygen atom, and R represents a group chosen from
| | |
|---|---|
| **R1 =** | 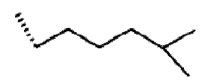 |
| **R2 =** | 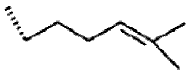 |
| **R3 =** | 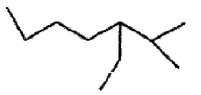 |
| **R4=** | 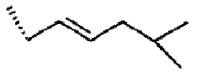 |
| **R6=** | 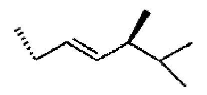 |
| **R5=** | 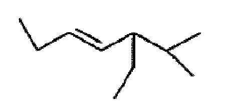 |
A represents a hydrogen atom or together with B a carbon-carbon bond,
B represents a hydrogen atom, a hydroxy group or together with A a carbon-carbon bond,
C represents a hydrogen atom or together with D a carbon-carbon bond,
D represents a hydrogen atom or together with C a carbon-carbon bond,
E represents a hydrogen atom or together with F a carbon-carbon bond,
F represents a hydrogen atom or together with E a carbon-carbon bond,
or one of its addition salts with pharmaceutically acceptable acids, for obtaining a medicament intended to be used as a neuroprotector.

8. Use according to claim 7, **characterized in that** the neuroprotective medicament is intended for the treatment of a neurodegenerative disease.

9. Use according to claim 7 or 8, **characterized in that** the neuroprotective medicament is intended for the treatment of a neurodegenerative disease chosen from Huntington's chorea, chronic neurodegenerative diseases, hereditary or sporadic, neuronal lesions linked with ageing; peripheral neuropathies that are hereditary or result from a lesion, Charcot-Marie-Tooth disease, diabetic neuropathies or those induced by anti-cancer treatments, traumatisms of the brain, the peripheral nerves or the spinal cord, ischemias of the brain or the spinal cord, degenerations which are hereditary, result from a lesion or linked with ageing of the sensory neurones of vision or degenerations of the optic nerve, degenerations which are hereditary, traumatic or linked with ageing of the sensory neurones of hearing, lobar atrophies and vascular dementias, diseases and traumatisms linked to degeneration of the motor neurones, spinal amyotrophies, multiple sclerosis and traumatisms of the spinal cord or the peripheral motor nerves.

10. Use according to one of claims 7 to 9, **characterized in that** the neuroprotective medicament is intended for the treatment of a neurodegenerative disease chosen from the pathologies or traumatisms linked to the degeneration or death of the neurons, in mammals suffering from such pathologies or traumatisms.

11. Use according to one of claims 7 to 10, **characterized in that** the neuroprotective medicament is intended for the treatment of infantile spinal amyotrophies.

12. Use according to one of claims 7 to 10, **characterized in that** the neuroprotective medicament is intended for the treatment of amyotrophic lateral sclerosis.

13. Use according to claim 7, **characterized in that** the neuroprotective medicament is intended for the treatment of painful sensitivity or chronic neuropathic pain.

14. Use according to claim 7, **characterized in that** the neuroprotective medicament is intended for the treatment of traumatic lesions of the nervous system.

15. Use according to claim 7, **characterized in that** the neuroprotective medicament is intended for the treatment of steroid-sensitive epilepsies, sleep disorders or alcohol intoxication.

16. Use according to claim 7, **characterized in that** the neuroprotective medicament is intended for the treatment of cognitive learning or memory disorders.

17. Use according to claim 7, **characterized in that** the neuroprotective medicament is intended for the treatment of anxiety or depression.

18. Use according to one of claims 7 to 17, **characterized in that** the compound of formula I according to claim 1 is the oxime of cholest-4-en-3-one.

19. A compound corresponding to formula I 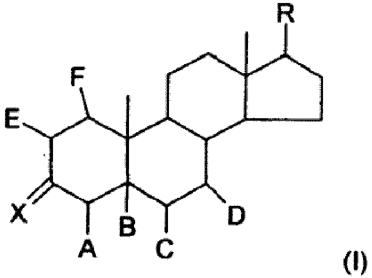 in which X represents an =N-OH radical and
- A together with B represents a carbon-carbon bond, C, D represent a hydrogen atom, E together with F represents a carbon-carbon bond and R has the meaning R1, or
- A together with B represents a carbon-carbon bond, C, D represent a hydrogen atom, E, F represent a hydrogen atom and R has the meaning R2 or R4, or
- A together with B represents a carbon-carbon bond, C represents together with D a carbon-carbon bond, E, F represent a hydrogen atom and R has the meaning R6, or
- A together with B represents a carbon-carbon bond, C represents together with D a carbon-carbon bond, E together with F represents a carbon-carbon bond and R has the meaning R1,
as well as its addition salts with mineral or organic acids.

20. A pharmaceutical composition, **characterized in that** it contains at least one of the medicaments as defined in claim 1 as active ingredient, as well as a pharmaceutically acceptable excipient.

21. A pharmaceutical composition, **characterized in that** it contains at least one of the medicaments as defined in claim 2 as active ingredient, as well as a pharmaceutically acceptable excipient.

22. A pharmaceutical composition, **characterized in that** it contains at least one of the medicaments as defined in any one of claims 3 to 6 as active ingredient, as well as a pharmaceutically acceptable excipient.

23. A pharmaceutical composition according to any one of claims 21 to 22, **characterized in that** the pharmaceutically acceptable excipient is at least one vegetable or animal oil.

## Patentansprüche

1. Verbindung gemäß Formel I 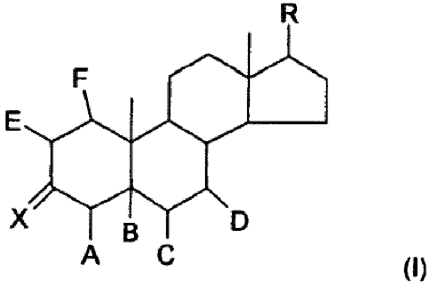 bei der X eine Gruppe = N-OH repräsentiert,
R eine Gruppe repräsentiert, die ausgewählt ist aus
| | |
|---|---|
| **R1 =** | 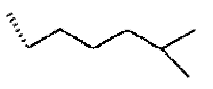 |
| **R2=** | 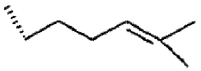 |
| **R3=** | 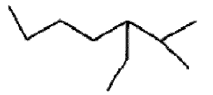 |
| **R4=** | 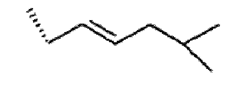 |
| **R6=** | 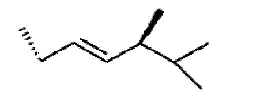 |
| **R5=** | 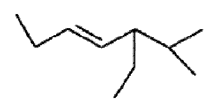 |
A ein Wasserstoffatom oder zusammen mit B eine Kohlenstoff-Kohlenstoff-Bindung repräsentiert,
B ein Wasserstoffatom, eine Hydroxygruppe oder zusammen mit A eine Kohlenstoff-Kohlenstoff-Bindung repräsentiert,
C ein Wasserstoffatom oder zusammen mit D eine Kohlenstoff-Kohlenstoff-Bindung repräsentiert,
D ein Wasserstoffatom oder zusammen mit C eine Kohlenstoff-Kohlenstoff-Bindung repräsentiert,
E ein Wasserstoffatom oder zusammen mit F eine Kohlenstoff-Kohlenstoff-Bindung repräsentiert,
F ein Wasserstoffatom oder zusammen mit E eine Kohlenstoff-Kohlenstoff-Bindung repräsentiert,
oder eines seiner Additionssalze mit den pharmazeutisch akzeptablen Säuren, zur Verwendung in einem Verfahren zur therapeutischen Behandlung eines menschlichen oder tierischen Körpers, d.h. als Medikament.

2. Verbindung nach Anspruch 1 zur Verwendung nach Anspruch 1, **dadurch gekennzeichnet, dass** in Formel I A zusammen mit B eine Kohlenstoff-Kohlenstoff-Bindung repräsentiert, C, D ein Wasserstoffatom repräsentieren, E, F ein Wasserstoffatom oder zusammen eine Kohlenstoff-Kohlenstoff-Bindung repräsentieren und R die Bedeutung R1 hat, d.h. das Oxim von Cholest-4-en-3-on oder das Oxim von 1,4-Cholestadien-3-on, oder eines seiner Additionssalze mit den pharmazeutisch akzeptablen Säuren, zur Verwendung als Medikament.

3. Verbindung nach Anspruch 1 zur Verwendung nach Anspruch 1, **dadurch gekennzeichnet, dass** in Formel I A zusammen mit B eine Kohlenstoff-Kohlenstoff-Bindung repräsentiert, C, D ein Wasserstoffatom repräsentieren, E, F ein Wasserstoffatom repräsentieren und R die Bedeutung R2 oder R3 oder R4 hat, oder eines seiner Additionssalze mit den pharmazeutisch akzeptablen Säuren, zur Verwendung als Medikament.

4. Verbindung nach Anspruch 1 zur Verwendung nach Anspruch 1, **dadurch gekennzeichnet, dass** in Formel I A zusammen mit B eine Doppelbindung repräsentiert, C zusammen mit D eine Kohlenstoff-Kohlenstoff-Bindung repräsentiert, E, F ein Kohlenstoffatom repräsentieren und R die Bedeutung R1 oder R6 hat, oder eines seiner Additionssalze mit den pharmazeutisch akzeptablen Säuren, zur Verwendung als Medikament.

5. Verbindung nach Anspruch 1 zur Verwendung nach Anspruch 1, **dadurch gekennzeichnet, dass** in Formel I A zusammen mit B eine Kohlenstoff-Kohlenstoff-Bindung repräsentiert, C zusammen mit D eine Kohlenstoff-Kohlenstoff-Bindung repräsentiert, E zusammen mit F eine Kohlenstoff-Kohlenstoff-Bindung repräsentiert und R die Bedeutung R1 hat, oder eines seiner Additionssalze mit den pharmazeutisch akzeptablen Säuren, zur Verwendung als Medikament.

6. Verbindung nach Anspruch 1 zur Verwendung nach Anspruch 1, **dadurch gekennzeichnet, dass** in Formel I E zusammen mit F eine Kohlenstoff-Kohlenstoff-Bindung repräsentiert, C, D, A, B ein Wasserstoffatom repräsentieren und R die Bedeutung R1 hat, oder eines seiner Additionssalze mit den pharmazeutisch akzeptablen Säuren, zur Verwendung als Medikament.

7. Verwendung einer Verbindung der Formel I 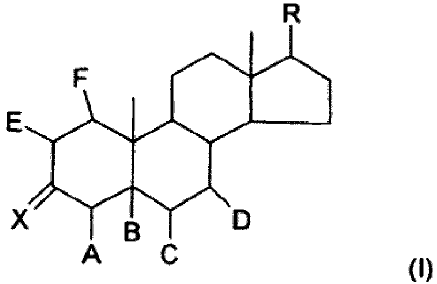 in der X eine Gruppe = N-OH oder ein Sauerstoffatom repräsentiert und R eine Gruppe repräsentiert, die ausgewählt ist aus
| | |
|---|---|
| **R1 =** | 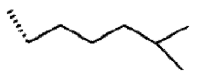 |
| **R2 =** | 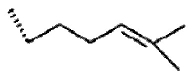 |
| **R3 =** | 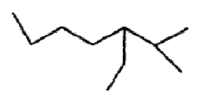 |
| **R4=** | 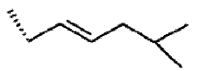 |
| **R6=** | 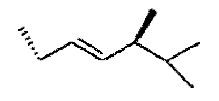 |
| **R5=** | 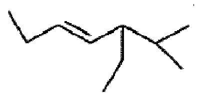 |
wobei A ein Wasserstoffatom oder zusammen mit B eine Kohlenstoff-Kohlenstoff-Bindung repräsentiert,
B ein Wasserstoffatom, eine Hydroxygruppe oder zusammen mit A eine Kohlenstoff-Kohlenstoff-Bindung repräsentiert,
C ein Wasserstoffatom oder zusammen mit D eine Kohlenstoff-Kohlenstoff-Bindung repräsentiert,
D ein Wasserstoffatom oder zusammen mit C eine Kohlenstoff-Kohlenstoff-Bindung repräsentiert,
E ein Wasserstoffatom oder zusammen mit F eine Kohlenstoff-Kohlenstoff-Bindung repräsentiert,
F ein Wasserstoffatom oder zusammen mit E eine Kohlenstoff-Kohlenstoff-Bindung repräsentiert,
oder eines seiner Additionssalze mit den pharmazeutisch akzeptablen Säuren, für den Erhalt eines Medikaments zur Verwendung als Neuroprotektor.

8. Verwendung nach Anspruch 7, **dadurch gekennzeichnet, dass** das Neuroprotektormedikament zur Behandlung einer neurodegenerativen Krankheit bestimmt ist.

9. Verwendung nach Anspruch 7 oder 8, **dadurch gekennzeichnet, dass** das Neuroprotektormedikament zur Behandlung einer neurodegenerativen Krankheit bestimmt ist, die ausgewählt ist aus der Huntington-Krankheit, chronischen neurodegenerativen, vererbbaren oder sporadischen Krankheiten, altersbedingten neuronalen Läsionen, peripheren vererbbaren oder läsionalen Neuropathien, der Charcot-Marie-Tooth-Krankheit, diabetischen oder durch Krebsbehandlung verursachten Neuropathien, Schäden an Gehirn, peripheren Nerven oder Rückenmark, Ischämien an Gehirn oder Rückenmark, vererbbarer, läsionaler oder altersbedingter Degeneration der sensorischen Neuronen des Sehens oder Degeneration des Sehnervs, vererbbarer, traumatischer oder altersbedingter Degeneration der sensorischen Neuronen des Gehörs, lobären Atrophien und vaskulären Demenzen, Erkrankungen und Schäden in Verbindung mit der Degeneration von Motorneuronen, spinalen Amyotrophien, multipler Sklerose und Schäden an Rückenmark oder peripheren motorischen Nerven.

10. Verwendung nach einem der Ansprüche 7 bis 9, **dadurch gekennzeichnet, dass** das Neuroprotektormedikament zur Behandlung einer neurodegenerativen Krankheit bestimmt ist, die ausgewählt ist aus Pathologien oder Schäden in Verbindung mit der Degeneration oder dem Absterben von Neuronen bei Säugetieren, die sich solche Pathologien oder Schäden zugezogen haben.

11. Verwendung nach einem der Ansprüche 7 bis 10, **dadurch gekennzeichnet, dass** das Neuroprotektormedikament zur Behandlung von infantilen spinalen Amyotrophien bestimmt ist.

12. Verwendung nach einem der Ansprüche 7 bis 10, **dadurch gekennzeichnet, dass** das Neuroprotektormedikament zur Behandlung von amyotrophen lateralen Sklerosen bestimmt ist.

13. Verwendung nach Anspruch 7, **dadurch gekennzeichnet, dass** das Neuroprotektormedikament zur Behandlung von Schmerzempfindlichkeit oder chronischen neuropathischen Schmerzen bestimmt ist.

14. Verwendung nach Anspruch 7, **dadurch gekennzeichnet, dass** das Neuroprotektormedikament zur Behandlung von traumatischen Läsionen des Nervensystems bestimmt ist.

15. Verwendung nach Anspruch 7, **dadurch gekennzeichnet, dass** das Neuroprotektormedikament zur Behandlung von steroidempfindlichen Epilepsien, Schlafbeschwerden oder Alkoholintoxikation bestimmt ist.

16. Verwendung nach Anspruch 7, **dadurch gekennzeichnet, dass** das Neuroprotektormedikament zur Behandlung von kognitiven Lernschwierigkeiten oder Gedächtnisstörungen bestimmt ist.

17. Verwendung nach Anspruch 7, **dadurch gekennzeichnet, dass** das Neuroprotektormedikament zur Behandlung von Angststörungen oder Depressionen bestimmt ist.

18. Verwendung nach einem der Ansprüche 7 bis 17, **dadurch gekennzeichnet, dass** die Verbindung von Formel I nach Anspruch 1 das Oxim von Cholest-4-en-3-on ist.

19. Verbindung gemäß Formel I 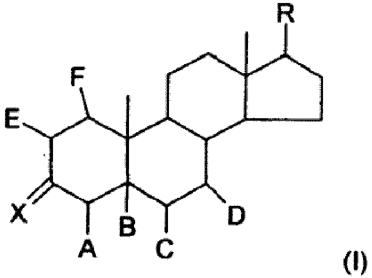 bei der X ein Radikal =N-OH repräsentiert, und
- A zusammen mit B eine Kohlenstoff-Kohlenstoff-Bindung repräsentiert, C, D ein Wasserstoffatom repräsentieren, E zusammen mit F eine Kohlenstoff-Kohlenstoff-Bindung repräsentiert und R die Bedeutung R1 hat, oder
- A zusammen mit B eine Kohlenstoff-Kohlenstoff-Bindung repräsentiert, C, D ein Wasserstoffatom repräsentieren, E, F ein Wasserstoffatom repräsentieren und R die Bedeutung R2 oder R4 hat, oder
- A zusammen mit B eine Kohlenstoff-Kohlenstoff-Bindung repräsentiert, C zusammen mit D eine Kohlenstoff-Kohlenstoff-Bindung repräsentiert, E, F ein Wasserstoffatom repräsentieren und R die Bedeutung R6 hat, oder
- A zusammen mit B eine Kohlenstoff-Kohlenstoff-Bindung repräsentiert, C zusammen mit D eine Kohlenstoff-Kohlenstoff-Bindung repräsentiert, E zusammen mit F eine Kohlenstoff-Kohlenstoff-Bindung repräsentiert und R die Bedeutung R1 hat, sowie ihre Additionssalze mit mineralischen oder organischen Säuren.

20. Pharmazeutische Zusammensetzung, **dadurch gekennzeichnet, dass** sie als Wirkstoff wenigstens eines der Medikamente wie in Anspruch 1 definiert sowie einen pharmazeutisch akzeptablen Exzipienten beinhaltet.

21. Pharmazeutische Zusammensetzung, **dadurch gekennzeichnet, dass** sie als Wirkstoff wenigstens eines der Medikamente wie in Anspruch 2 definiert sowie einen pharmazeutisch akzeptablen Exzipienten beinhaltet.

22. Pharmazeutische Zusammensetzung, **dadurch gekennzeichnet, dass** sie als Wirkstoff wenigstens eines der Medikamente wie in einem der Ansprüche 3 bis 6 definiert sowie einen pharmazeutisch akzeptablen Exzipienten beinhaltet.

23. Pharmazeutische Zusammensetzung nach einem der Ansprüche 21 bis 22, **dadurch gekennzeichnet, dass** der pharmazeutisch akzeptable Exzipient wenigstens ein pflanzliches oder tierisches Öl ist.
